(19) Europäisches Patentamt
European Patent Office
Office européen des brevets

(11) Veröffentlichungsnummer: **0 271 442 B1**

(12) **EUROPÄISCHE PATENTSCHRIFT**

(45) Veröffentlichungstag der Patentschrift: **30.09.92**

(51) Int. Cl.[5]: **C08G 59/32**, C08G 59/02

(21) Anmeldenummer: **87810700.2**

(22) Anmeldetag: **26.11.87**

Die Akte enthält technische Angaben, die nach dem Eingang der Anmeldung eingereicht wurden und die nicht in dieser Patentschrift enthalten sind.

# (54) Multifunktionelle Epoxidharze.

(30) Priorität: **02.12.86 CH 4801/86**

(43) Veröffentlichungstag der Anmeldung:
**15.06.88 Patentblatt 88/24**

(45) Bekanntmachung des Hinweises auf die Patenterteilung:
**30.09.92 Patentblatt 92/40**

(84) Benannte Vertragsstaaten:
**CH DE ES FR GB IT LI NL**

(56) Entgegenhaltungen:
**EP-A- 0 204 659**
**FR-A- 1 173 530**
**FR-A- 1 185 580**
**FR-A- 1 262 181**
**GB-A- 828 364**

**"Polymere Werkstoffe", H. Batzer, Bd. III, S. 171-172 (1984) G. Thieme Verlag**

(73) Patentinhaber: **CIBA-GEIGY AG**
**Klybeckstrasse 141**
**CH-4002 Basel(CH)**

(72) Erfinder: **Monnier, Charles E., Dr.**
**Ch. du Verger 16**
**CH-1752 Villars-sur-Glâne(CH)**
Erfinder: **Eldin, Sameer H., Dr.**
**Route de Schiffenen 10**
**CH-1700 Fribourg(CH)**
Erfinder: **Flury, Peter, Dr.**
**Muldenweg**
**CH-4249 Himmelried(CH)**

**EP 0 271 442 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft multifunktionelle Epoxidharze auf Basis von Biphenyl-, Diphenylmethan- und Diphenylsulfonderivaten, die entsprechenden Diallyldiglycidylether-Zwischenprodukte, Gemische enthaltend besagte multifunktionelle Epoxidharze und Härter sowie die Verwendung dieser härtbaren Gemische als Klebstoffe oder zur Herstellung von Verbundwerkstoffen und Laminaten.

Multifunktionelle Epoxidharze, die sich durch Epoxidierung von Allylphenylglycidylethern erhalten lassen, sind bekannt. So beschreibt die GB-PS 828,364 Ether von epoxi-substituierten Phenolen, beispielsweise 2,2-Bis-(4-epoxipropoxy-3-epoxipropylphenyl)-propan. Die Verbindung wird durch Epoxidierung von 2,2-Bis-(4-epoxipropoxy-3-allylphenyl)-propan mit Peressigsäure hergestellt.

In Adv. Chem. Ser., 92, 173-207 (1970) beschreiben A.L. Cupples et al. verschiedene Epoxidharze, die zur raschen Aushärtung in kleinen Mengen eingesetzt werden sollen. Unter anderem werden auch die Tetraglycidylether von Bis-(2,4-dihydroxyphenyl)-methan und von 2,2',4,4'-Tetrahydroxybiphenyl untersucht. Die Verbindungen werden als relativ langsam härtend eingestuft und dieses Verhalten wird auf die hohe Viskosität bei Raumtemperatur zurückgeführt.

In Polymer Bulletin 4, 479-486 (1981) untersuchen E.A. Dzvahadvan et al. den Einfluss der Brückengruppen auf die Reaktivität und die mechanischen Eigenschaften verschiedener Diglycidylether von 4,4'-bisaromatischen Verbindungen. Tetra- oder höher funktionelle Derivate werden nicht untersucht.

Im US-Patent 2,967,161 werden Bis-(allyl-glycidyloxyphenyl)-alkanverbindungenbeschrieben, die zusammen mit ungesättigten Polyamiden vernetzt werden können. Die Alkylenbrücke zwischen den Phenylresten muss zwei bis vier C-Atome besitzen.

Im GB-Patent 823,181 wird die Herstellung von epoxi-substituierten aromatischen Verbindungen,unter anderem von Biphenylderivaten, beschrieben. Tetra- oder höherfunktionelle Derivate werden nicht erwähnt. Die Verbindungen leiten sich von allylsubstituierten Aromaten ab und werden in der Regel durch Epoxidierung dieser Vorläufer mit Persäure gewonnen.

Es wurde jetzt eine ausgewählte Gruppe von multifunktionellen Epoxidharzen gefunden, die sich durch verbesserte mechanische und/oder rheologische Eigenschaften auszeichnen. So besitzen Mischungen dieser Harze mit Härtern in der Regel niedrige Ausgangsviskositäten, was ihre Verarbeitung erleichtert oder die Herstellung hochgefüllter Systeme ermöglicht. Die gehärteten Mischungen zeichnen sich durch hohe Biegefestigkeit, gute adhäsive Eigenschaften, insbesondere auf Metallen, und eine niedrige Wasseraufnahme aus.

Die vorliegende Erfindung betrifft Verbindungen der Formel I

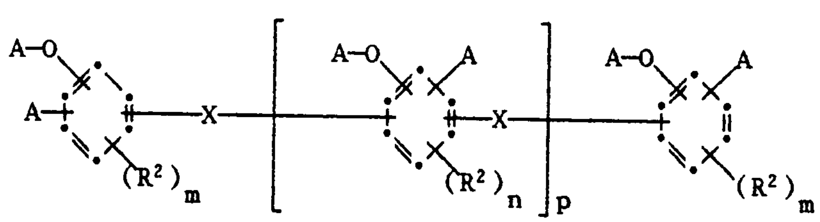 (I),

worin A eine Gruppe

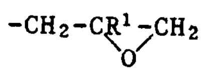

ist, $R^1$ Wasserstoff oder Methyl bedeutet, $R^2$ $C_1$-$C_6$ Alkyl, Halogen oder Phenyl ist,
X eine direkte C-C Bindung, $-CH_2-$ oder $-SO_2-$ darstellt,
m 0, 1, 2 oder 3 ist, n 0, 1 oder 2 bedeutet und
p 0 oder, im Falle von X = $-CH_2-$, auch eine ganze Zahl von 1 bis 6 bedeutet, mit der Massgabe, dass die Gruppen -A und -O-A jeweils in ortho-Position zueinander stehen.

$R^1$ ist vorzugsweise Wasserstoff.

$R^2$ als $C_1$-$C_6$ Alkyl ist geradkettig oder verzweigt, vorzugsweise geradkettig. Beispiele für solche Reste sind Methyl, Ethyl, n-Propyl, Isopropyl, n-Butyl, Isobutyl, n-Pentyl oder n-Hexyl. Bevorzugt wird Methyl.

$R^2$ als Halogen bedeutet Fluor, Chlor, Brom oder Iod, bevorzugt werden Chlor und Brom, insbesondere

Chlor.
Der Index m ist vorzugsweise 0 oder 1, ganz besonders bevorzugt jedoch 0.
Der Index n ist vorzugsweise 0 oder 1, ganz besonders bevorzugt jedoch 0.
Der Index p ist vorzugsweise 0.
Besonders bevorzugt werden Verbindungen der Formel I, worin m, n und p 0 sind.

Die Brückengruppe X ist vorzugsweise eine direkte C-C Bindung oder $-SO_2-$. Ganz besonders bevorzugt handelt es sich bei der Brücke um eine direkte C-C Bindung.

Besonders bevorzugt werden Verbindungen der Formel I, worin die Reste -O-A jeweils in ortho- oder para-Position zur Brücke -X-stehen.

Ganz besonders bevorzugt werden Verbindungen der Formel I, worin die Reste -O-A jeweils in ortho-Position zur Brücke -X- stehen.

Die Verbindungen der Formel I können durch Epoxidierung der entsprechenden Allyl- oder Methallylglycidylether mit Persäure erhalten werden. Diese Zwischenprodukte sind neu und fallen ebenfalls unter den Rahmen der vorliegenden Erfindung.

Die Erfindung betrifft daher auch Verbindungen der Formel II

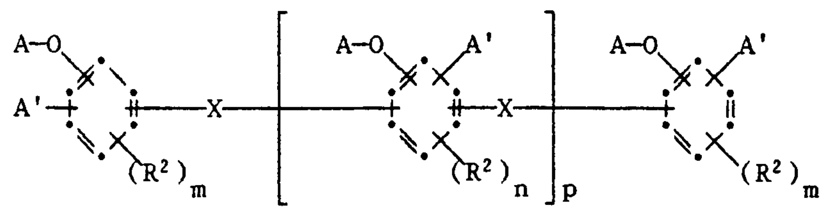 (II),

worin A eine Gruppe

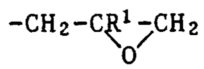

ist, $R^1$ Wasserstoff oder Methyl bedeutet, A' eine Gruppe $-CH_2-CR^1=CH_2$ ist, $R^2$ $C_1$-$C_6$Alkyl, Halogen oder Phenyl ist,
X eine direkte C-C Bindung, $-CH_2-$ oder $-SO_2-$ darstellt,
m 0, 1, 2 oder 3 ist, n 0, 1 oder 2 bedeutet und
p 0 oder, im Falle von X = $-CH_2-$, auch eine ganze Zahl von 1 bis 6 bedeutet, mit der Massgabe, dass die Gruppen -A' und -O-A jeweils in ortho-Position zueinander stehen.

Die Verbindungen der Formel II werden in an sich bekannter Weise durch Umsetzung der entsprechenden Allylphenole mit Epihalogenhydrin oder $\beta$-Methylepihalogenhydrin, insbesondere mit Epichlorhydrin, hergestellt.

Die Allylphenole werden in an sich bekannter Weise durch Veretherung der entsprechenden Phenole und anschliessende Claisen Umlagerung hergestellt. Beispiele für solche Reaktionen sind in der EP-A 13,258 beschrieben.

Die als Ausgangsmaterialien zur Herstellung der Verbindungen der Formel II eingesetzten Polyphenole sind bekannt und grösstenteils im Handel erhältlich. Beispiele für solche Verbindungen sind 4,4'-Dihydroxybiphenyl, 4,4'-Dihydroxydiphenylmethan, 4,4'-Dihydroxydiphenylsulfon oder Novolake auf Basis von Phenol und Formaldehyd. Als Persäuren für die Epoxidierung der veretherten Verbindungen II kommen vor allem organische Persäuren, wie z.B. Perameisensäure, Peressigsäure, Perbernsteinsäure, Perbenzoesäure, m-Chlorperbenzoesäure und Monoperphthalsäure, in Betracht. Die organischen Persäuren können als solche eingesetzt oder in situ gebildet werden, beispielsweise aus aliphatischen oder aromatischen Carbonsäuren, Carbonsäureanhydriden, Carbonsäureestern, Säurechloriden oder Keten und Wasserstoffperoxid. Zur in-situ-Bildung der Persäuren verwendet man vorzugsweise aliphatische oder aromatische Mono- oder Dicarbonsäuren oder deren Anhydride, wie Ameisensäure, Essigsäure, Propionsäure, Bernsteinsäureanhydrid, Benzoesäure, oder Phthalsäure, und Wasserstoffperoxid, gegebenenfalls unter Zusatz von sauren Katalysatoren, wie Schwefelsäure oder Alkalimetallsalzen. Die Epoxidierung der Addukte wird bevorzugt in Gegenwart von vorgebildeter oder in situ erzeugter Perameisensäure oder Peressigsäure durchgeführt. Gewünschtenfalls können auch anorganische Persäuren, wie Permolybdänsäure, Pervanadinsäure oder

Perwolframsäure, eingesetzt werden. Das Epoxidierungsmittel (Persäure) wird zweckmässig in einer Menge von mindestens 1 Mol pro vorhandene (Meth)allylgruppe und vorzugsweise im Ueberschuss, z.B. einem 20-200%igen molaren Ueberschuss, verwendet.

Die Veretherung der Ausgangsphenole bzw. der Allylphenole und die Epoxidierung der Produkte wird mit Vorteil in Gegenwart inerter organischer Lösungsmittel und im Falle der Epoxidierung, gegebenenfalls unter Zusatz von Puffersubstanzen, wie Natriumacetat oder Natriumhydrogenphosphat, vorgenommen. Als Lösungsmittel eignen sich z.B. gegebenenfalls halogenierte aliphatische oder aromatische Kohlenwasserstoffe, wie Chloroform, Dichlormethan, Benzol, Toluol und Chlorbenzol, Ether, wie Diethylether, Diisopropylether, Dioxan und Tetrahydrofuran, sowie Carbonsäurealkylester, wie Essigsäureethylester und -n-butylester. Halogenierte, besonders chlorierte aliphatische Kohlenwasserstoffe sind als Lösungsmittel bevorzugt; besonders bevorzugt ist Chloroform. Die Reaktionstemperaturen liegen im allgemeinen zwischen -10 und +100 °C, bevorzugt zwischen 10 und 60 °C.

Eine weitere Herstellungsmethode der Verbindungen der Formel I geht von den entsprechenden polyallylierten Bisphenolen aus. Durch Epoxidierung mit Persäuren werden anschliessend die kernständigen Allylgruppen und die Allyloxygruppen epoxidiert. Beispiele für solche Umsetzungen sind in der JP-OS 59-124,905 gegeben. Bei dieser Verfahrensvariante entstehen besonders halogenarme Derivate.

Die erfindungsgemäss erhältlichen Polyepoxide sind reine Substanzen, die im wesentlichen frei von Chlorid- und Alkalimetallionen sind. Sie eignen sich zur Herstellung von gehärteten Produkten, insbesondere als Matrixharze für Verbundwerkstoffe.

Weiterer Gegenstand der Erfindung sind daher Gemische enthaltend

a) ein Polyepoxid der Formel I und

b) einen Härter für die Komponente a).

Dabei können auch Gemische verschiedener erfindungsgemäss erhältlicher Polyepoxide und/oder Härter verwendet werden. Als Härter b) eignen sich im allgemeinen beliebige Epoxidharzhärter, wie z.B. Cyanamid, Dicyandiamid, Polycarbonsäuren, Polycarbonsäureanhydride, Polyamine, Polyaminoamide, Addukte aus Aminen und Polyepoxiden und Polyole.

Geeignete Polycarbonsäuren und ihre Anhydride sind z.B. Phthalsäureanhydrid oder Tetrahydro- und Hexahydrophthalsäureanhydrid, sowie die zu den oben genannten Anhydriden gehörenden Säuren.

Als Beispiele von Polyaminen, die sich als Härtungsmittel eignen, seien aliphatische, cycloaliphatische, aromatische und heterocyclische Polyamine, wie Hexamethylendiamin, Diethylentriamin, m-Xylylendiamin, Bis(4-amino-cyclohexyl)methan, m- und p-Phenylendiamin, Bis (4-aminophenyl)methan, Bis(4-aminophenyl)-sulfon und Anilin-Formaldehydharze genannt. Geeignete Polyaminoamide sind z.B. solche, die aus aliphatischen Polyaminen und dimerisierten oder trimerisierten ungesättigten Fettsäuren hergestellt sind.

Als Polyolhärter b) kommen vor allem ein- oder mehrkernige aromatische Polyole, einschliesslich Novolake, in Betracht, wie Resorcin, Hydrochinon, 2,6-Dihydroxytoluol, Pyrogallol, 1,1,3-Tris-(hydroxyphenyl)propan, Bis(4-hydroxyphenyl)methan, 2,2-Bis(4-hydroxyphenyl)propan, Bis(4-hydroxyphenyl)sulfon und 4,4′-Dihydroxybiphenyl sowie Novolake aus Formaldehyd oder Acetaldehyd und Phenol, Chlorphenol oder Alkylphenolen mit bis zu 9 C-Atomen im Alkyl, besonders Kresol- und Phenolnovolake.

Bevorzugte Härter sind Polycarbonsäureanhydride, wie Tetrahydro-, Hexahydrophthalsäureanhydrid, sowie aromatische Polyamine, besonders Bis(4-aminophenyl)methan, Bis(4-aminophenyl)sulfon und m- oder p-Phenylendiamin, sowie ganz besonders Polyolhärter auf Novolakbasis, insbesondere Kresolnovolake oder Phenolnovolake.

Die erfindungsgemässen Gemische können auch weitere übliche Zusätze enthalten, vor allem c) einen Beschleuniger und/oder d) weitere Epoxidharze.

Als Beschleuniger c) können ebenfalls an sich bekannte Verbindungen verwendet werden. Als Beispiele seien genannt: Komplexe von Aminen, besonders tertiären Aminen, wie Monoethylamin mit Bortrifluorid oder Bortrichlorid, tertiäre Amine, wie Benzyldimethylamin; Harnstoffderivate, wie N-4-Chlorphenyl-N′,N′-dimethylharnstoff (Monuron); gegebenenfalls substituierte Imidazole, wie Imidazol oder 2-Phenylimidazol. Tertiäre Amine, besonders Benzyldimethylamin, und Imidazole, besonders 2-Phenylimidazol oder 2-Ethyl-4-methylimidazol, sind als Beschleuniger c) bevorzugt.

Als weitere Epoxidharze d) kommen vor allem solche mit durchschnittlich mehr als einer an ein Heteroatom, z.B. an ein S- und vorzugsweise an ein O- oder N-Atom, gebundenen Gruppen A, wie sie im Vorangehenden beschrieben sind, in Betracht.

Besonders bevorzugt setzt man als Komponente d) gegebenenfalls vorverlängerte Diglycidylether von zweiwertigen Phenolen oder Cyclohexanolen, vor allem 2,2-Bis(4-hydroxyphenyl)propan, 2,2-Bis-(dibrom-4-hydroxyphenyl)-propan, Bis(4-hydroxyphenyl)methan, Bis(4-hydroxycyclohexyl)methan oder 2,2-Bis(4-hydroxycyclohexyl)-propan, Polyglycidylether von Novolaken, oder tetraglycidyliertes 4,4′-Diaminodiphenylmethan ein. Ganz besonders bevorzugt sind gegebenenfalls vorverlängerte Diglycidylether von Bisphenol A,

Tetrabrom-bisphenol A oder Bisphenol F, Polyglycidylether von Phenol-Formaldehyd- oder Kresol-Formaldehyd-Novolaken, oder Gemische davon.

Die Komponenten b) und c) werden in den üblichen wirksamen, d.h. für die Härtung der erfindungsgemässen Gemische ausreichenden Mengen eingesetzt. Das Verhältnis der Komponenten a), b), c) und gegebenenfalls d) hängt von der Art der verwendeten Verbindungen, der erforderlichen Härtungsgeschwindigkeit und den im Endprodukt gewünschten Eigenschaften ab und kann vom Fachmann auf dem Gebiet der Epoxidharz-Härtung leicht ermittelt werden. Wenn das Härtungsmittel b) ein Amin ist, werden normalerweise 0,75 bis 1,25 Aequivalent Aminwasserstoff pro 1 Epoxidäquivalent eingesetzt. Bei Polycarbonsäure- oder Polycarbonsäureanhydrid-Härtern verwendet man gewöhnlich 0,4 bis 1,1 Aequivalente Carboxyl- bzw. Anhydridgruppen pro 1 Epoxidäquivalent. Bei der Verwendung von Polyphenolen als Härtungsmittel setzt man zweckmässig 0,75 bis 1,25 phenolische Hydroxylgruppen pro 1 Epoxidäquivalent ein. Beschleuniger c) werden im allgemeinen in Mengen von 0,1 bis 5 Gewichtsprozent, bezogen die Epoxidharze a) und gegebenenfalls d), verwendet.

Gewünschtenfalls kann man den härtbaren Gemischen zur Herabsetzung der Viskosität reaktive Verdünner, wie z.B. Styroloxid, Butylglycidylether, 2,2,4-Trimethylpentylglycidylether, Phenylglycidylether, Kresylglycidylether oder Glycidylester von synthetischen, hochverzweigten, in der Hauptsache tertiären aliphatischen Monocarbonsäuren, zusetzen. Als weitere übliche Zusätze können die erfindungswgemässen Gemische ferner Weichmacher, Streck-, Füll- und Verstärkungsmittel, wie beispielsweise Steinkohlenteer, Bitumen, Texilfasern, Glasfasern, Asbestfasern, Borfasern, Kohlenstoff-Fasern, mineralische Silikate, Glimmer, Quarzmehl, Aluminiumoxidhydrat, Bentonite, Kaolin, Kieselsäureaerogel oder Metallpulver, z.B. Aluminiumpulver oder Eisenpulver, ferner Pigmente und Farbstoffe, wie Russ, Oxidfarben und Titandioxid, Flammschutzmittel, Thixotropiemittel, Verlaufmittel ("flow control agents"), wie Silicone, Wachse und Stearate, die zum Teil auch als Formtrennmittel Anwendung finden, Haftvermittler, Antioxidantien und Lichtschutzmittel enthalten.

Die erfindungsgemässen Gemische finden z.B. Anwendung als Klebstoffe oder zur Herstellung von gehärteten Produkten, wie Verbundwerkstoffen und Laminaten, insbesondere jedoch als Matrixharze für Verbundwerkstoffe. Sie können in jeweils dem speziellen Anwendungsgebiet angepasster Formulierung, in ungefülltem oder gefülltem Zustand, z.B. als Anstrichmittel, Beschichtungsmassen, Lacke, Pressmassen, Tauchharze, Giessharze, Imprägnierharze, Laminierharze Matrixharze und Klebemittel verwendet werden.

Die Härtung der erfindungsgemässen Gemische kann auf an sich bekannte Weise ein- oder zweistufig vorgenommen werden. Die Härtung der erfindungsgemässen Gemische erfolgt im allgemeinen durch Erhitzen auf Temperaturen zwischen 80 und 200 °C, besonders 100 und 180 °C.

Die mit den erfindungsgemässen Polyepoxiden hergestellten gehärteten Produkte zeichnen sich durch gute mechanische, thermische, elektrische und chemische Eigenschaften aus.

Die Erfindung wird durch die folgenden Beispiele näher erläutert. Teile bedeuten Gewichtsteile.

A) Herstellung der Zwischenprodukte

A1) 3,3'-Diallylbiphenyl-4,4'-diglycidylether

a) 3,3'-Diallyl-4,4'-dihydroxybiphenyl

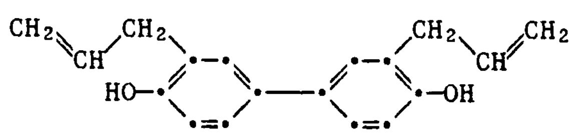

In einem 50 ml Rundkolben mit Kühler, Thermometer und Rührer werden 26,63 g (0,1 Mol) Biphenyl-4,4'-diallylether gelöst in 27 ml Toluol, 0,11 g Natriumcarbonat und 0,43 g LiCl vorgelegt und auf 188-192 °C erwärmt. Dabei destilliert das Toluol ab. Nach 2 Stunden Reaktionszeit wird das Reaktionsgemisch abgekühlt, in Chloroform aufgenommen und mit Wasser gewaschen. Die organische Phase wird getrocknet und eingeengt wobei 20,50 g (77 %) 3,3'-Diallyl-4,4'-dihydroxybiphenyl isoliert werden.

NMR ($CDCl_3$) 3,5 d 4H (Allyl-$CH_2$), 5,0-5,3 m 4H ($\underline{CH_2}$=CH), 5,8-6,25 m 2H ($CH_2$=$\underline{CH}$-), 6,75-7,4 d x d 6H (arom. H).

IR:KBr 3000-3200 br. (-OH), 1630, 1610, 1500, 1400, 1240 $cm^{-1}$.

b) 3,3'-Diallylbiphenyl-4,4'-diglycidylether

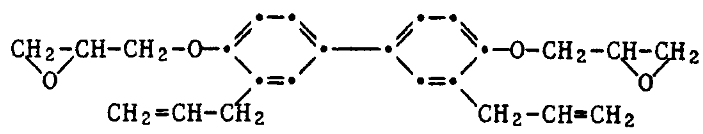

In einem 750 ml Sulfierkolben mit Kühler, Thermometer und Rührer werden 53,27 g (0,200 Mol) 3,3'-Diallyl-4,4'-dihydroxybiphenyl, 272,70 g (2,93 Mol) Epichlorhydrin und 3,44 g Tetramethylammoniumchlorid vorgelegt und auf 115 °C während 4h erwärmt. Es entsteht eine klare, gelbliche Lösung.

Anschliessend wird das Reaktionsgemisch abgekühlt und bei 45-60 °C werden unter gleichzeitigem azeotropem Abdestillieren 37,90 g (0,471 Mol) 50%ige Natronlauge zugetropft. Das Reaktionsgemisch wird so lange reagieren gelassen, bis sich kein Wasser mehr abspaltet. Nach Beendigung wird das Reaktionsgemisch filtriert, mit ca. 200 ml Wasser und 1 N HCl neutral gestellt, über $Na_2SO_4$ getrocknet, filtriert und eingeengt. Es resultieren 77,3 g 3,3'-Diallylbiphenyl-4,4'-diglycidylether vom Epoxidgehalt 5,02 Aequ./kg (95%) und einer Viskosität von 1520 mPas/25 °C.

IR (Film) 3000-3300 schwach, 3030-2800, 1640, 1600, 1500, 1340 $cm^{-1}$.

NMR ($CDCl_3$) 2,75-3,0 m 4H (-$CH_2$CH$\underline{CH_2}$ (O)), 3,25-3,5 m 4H (-$\underline{CH_2}$CH=$CH_2$)

3,75-4,3 m 6H (-$\underline{CH_2CH}$CH$_2$ (O)), 4,75-5,25 m 4H (-$CH_2$CH=$\underline{CH_2}$)

5,75-6,25 m 2H ($CH_2\underline{CH}$=$CH_2$), 6,75-7,50 m 6H (aromat. H)

A2) 3,3'-Diallyldiphenylmethan-4,4'-diglycidylether

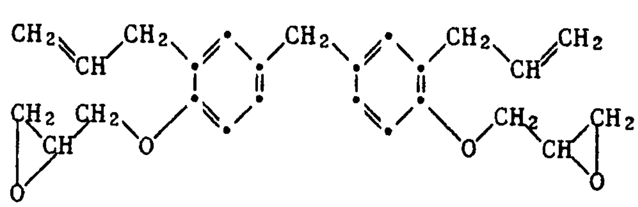

In einem 1,5 ℓ Sulfierkolben mit Rührer, Kühler und Tropftrichter werden 114,47 g (0,408 Mol) 3,3'-Diallylbisphenol-F (para, para), 7,04 g Tetramethylammoniumchlorid und 556,30 g (5,967 Mol) Epichlorhydrin vorgelegt und während 4 Stunden auf 115 °C erwärmt.

Anschliessend wird das Reaktionsgemisch auf 60 °C abgekühlt. Bei dieser Temperatur werden 77,30 g (0,960 Mol) 50%ige Natronlauge innerhalb ca. 3 Stunden zugetropft, wobei das entstehende Reaktionswasser unter Vakuum abdestilliert wird. Nach Beendigen wird das Reaktionsgemisch filtriert, das Filtrat mit 2 x

250 ml Wasser ausgewaschen, über $Na_2SO_4$ getrocknet, filtriert und eingeengt. Es resultieren 154,59 g (96,55 % d.Th.) eines gelben, klaren Oels der Viskosität 394 mPas/25 °C und vom Epoxidgehalt 4,48 Aequ./kg (87,96 % d.Th.)

IR (Film) 3000-3300 schwach, 3080-2800, 1640, 1610, 1500, 1250, 1170, 1030, 920 $cm^{-1}$

$\overline{M}_n$ 412; $\overline{M}_w$ 426.

A3) 3,3'-Diallyldiphenylmethan-2,2'-diglycidylether

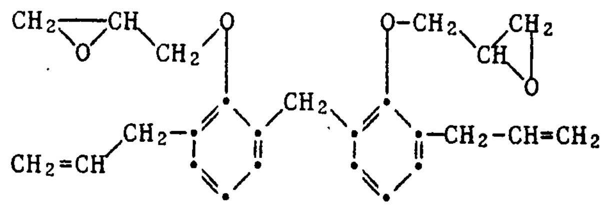

In einem 1,5 ℓ Sulfierkolben mit Rührer, Kühler, Thermometer und Tropftrichter werden 140,18 g (0,5 Mol) o,o'-Diallyl-bisphenol-F, 681,14 g (6,74 Mol) Epichlorhydrin und 8,04 g Tetramethylammoniumchlorid 50 % vorgelegt und während 4 Stunden auf 111 °C erwärmt. Anschliessend wird das Reaktionsgemisch auf 52-56 °C abgekühlt und innerhalb 3 Stunden werden 94,67 g (1,18 Mol) 50%ige Natronlauge zugetropft, unter gleichzeitigem azeotropem Auskreisen von Wasser. Nach Beendigen des Auskreisens wird das Reaktionsgemisch über einem Seitzfilter filtriert, anschliessend wird das Filtrat mit Wasser und 1N HCl neutral gewaschen, über $Na_2SO_4$ getrocknet und eingeengt, wobei 184,77 g (94,15 % d.Th.), 3,3'-Diallyldiphenylmethan-2,2'-diglycidylether vom Epoxidgehalt 4,212 Aequ./kg (82,66 % d.Th) und einer Viskosität von 395 mPas/25 °C erhalten werden.

IR (Film) 3500-3300 (OH), 1640, 1450, 1250, 1080, 1020, 910 $cm^{-1}$

$\overline{M}_n$ 369; $\overline{M}_w$ 382.

A4) 3,3'-Dialyldiphenylsulfon-4,4'-diglycidylether

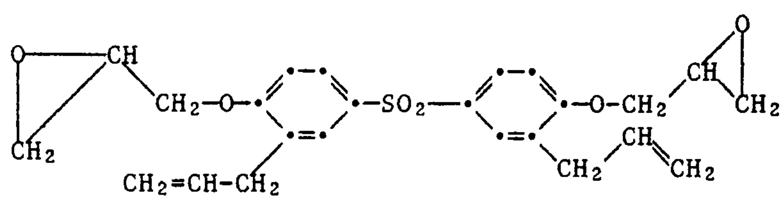

In einem 2,5 ℓ Sulfierkolben mit Wasserabscheider, Rührer, Vakuumaufsatz, Thermometer und Tropftrichter werden 300,00 g (0,91 Mol) 3,3'-Diallyl-4,4'-dihydroxydiphenylsulfon und 1238,37 g (13,28 Mol) Epichlorhydrin vorgelegt und auf 60 °C erwärmt. Anschliessend werden 15,65 g 50 % Tetramethylammoniumchloridlösung hinzugefügt und das Gemisch wird auf 110-112 °C während 4 Stunden erwärmt. Das Reaktionsgemisch auf 50-55 °C abgekühlt und unter gleichzeitigem azeotropem Abdestillieren des Reaktionswassers werden 172,10 g (2,13 Mol) 50%ige Natronlauge zugetropft. Das Reaktionsgemisch wird solange ausreagieren gelassen, bis sich kein Wasser mehr abscheidet. Anschliessend wird das Reaktionsgemisch filtriert, das Filtrat mit 5 % HCl neutral gewaschen, über $Na_2SO_4$ getrocknet und bei 60 °C eingeengt. Es resultieren 400,60 g (99,7 % d.Th.) eines viskosen Harzes ($\eta$ 40 °C = 2410 mPas) vom Epoxidgehalt 3,581 Aeq./kg (79,23 %)

IR (Film) 3500-3300, 3080-2900, 1640, 1650, 1490, 1310, 1250, 1150-1100, 1020, 920, 830 $cm^{-1}$.

NMR($CDCl_3$) 2,5-4,5 m ~ 10H ($\overset{O}{\overline{CH_2CH}}CH_2-$), 4,2-6,0 m 10H (Allyl-), 6,7-8,0 m 6H (Aryl).

$\overline{M}_n$ 420; $\overline{M}_w$ 433.

A5) 3,3-Diallylbiphenyl-2,2'-diglycidylether

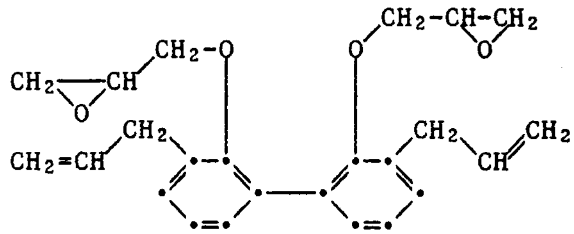

In einem 1,5 ℓ Sulfierkolben mit Rührer, Kühler und Tropftrichter werden 151,83 g (0.57 Mol) 3,3'-Diallyl-2,2'-dihydroxybiphenyl, 777,20 g (8,34 Mol) Epichlorhydrin und 9,80 g 50%ige Tetramethylammoniumchloridlösung vorgelegt und auf 118 °C erwärmt. Nach 4 Stunden Reaktionszeit wird das Reaktionsgemisch auf 60 °C abgekühlt und unter Vakuum und gleichzeitigem Auskreisen des Reaktionswassers werden innerhalb einer Stunde 108,02 g (1,34 Mol) 50%ige Natronlauge zugetropft. Das Reaktionsgemisch wird noch während etwa 3 Stunden ausreagieren gelassen. Anschliessend wird das gebildete Salz über Filtrierhilfe filtriert und mit Toluol verdünnt, dreimal mit Wasser und $NaHSO_4$-Lösung gewaschen, über $Na_2SO_4$ getrocknet und eingeengt. Es resultieren 197,7 g (91,63 % d.Th.) eines dünnflüssigen Harzes ($\eta_{25}$: 560 mPas) vom Epoxidgehalt 4,061 Aeq/kg.

IR (Film) 3500-3300 (br.), 3150-2860, 1640, 1440, 1200, 1210, 1020, 910, 840, 760 $cm^{-1}$.

NMR($CDCl_3$) 2,2-2,6 m, 4H (-O$\underline{CH_2}$CH), 2,8-3,0 m 2H ($\underline{CH}$-$CH_2$), 3,5-4,5 m 8H ($\underline{CH_2}$-CH=$CH_2$ und O-$CH_2$-), 5,0-5,2 m 4H ($\underline{CH_2}$=CH); 5,8-6,2 m 2H (-$\underline{CH}$=$CH_2$); 6,8-7,2 m 6H (arom. H)

$\overline{M}_n$ 352; $\overline{M}_w$ 359.

B) Herstellung der Tetraglycidylverbindungen

B1) 3,3'-Diglycidylbiphenyl-4,4'-diglycidylether

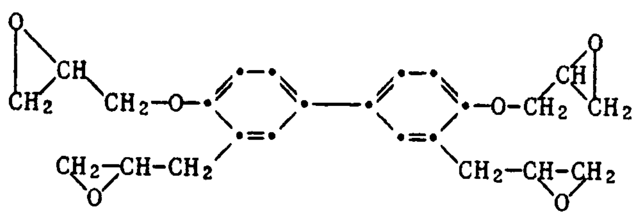

In einem 350 mol Sulfierkolben mit Rührer, Kühler, Thermometer und Tropftrichter werden 70,00 g (0,185 Mol), 3,3'-Diallylbiphenyl-4,4'-diglycidylether (Beispiel A1), 2,99 g Natriumacetat in 20 ml Chloroform vorgelegt. Bei Raumtemperatur werden innerhalb ca. 2 Stunden 80,56 g (0,39 Mol) 40%ige Peressigsäure zugetropft. Nach Beenden der Zugabe wird das Reaktionsgemisch noch während 3 Stunden gerührt, anschliessend mit 200 ml Chloroform versetzt und die organische Phase mehrmals mit Wasser neutral gewaschen, über $Na_2SO_4$ getrocknet, filtriert und am Vakuum eingeengt. Es resultieren 70,45 g (92,77 % d.Th.), 3,3'-Diglycidylbiphenyl-4,4'-diglycidylether vom Epoxidgehalt 8,230 Aequ./kg (84,47 % d.Th.) und der Viskosität 280 mPas/80 °C.

IR (Film) 3500-3300, 3030-2800, 1740, 1610, 1470, 1230, 1130, 1020 $cm^{-1}$

NMR($CDCl_3$) 2,5-3,0 m ca. 8H ($CH_2$-CH und -$CH_2$-C); 3,1-3,5 m 4 H (CH-$CH_2$) 4,0-4,5 m ca. 4 H (O-$CH_2$-CH) 6,75-7,5 m 6H (arom. H).

B2) 3,3'-Diglycidyldiphenylmethan-4,4'-diglycidylether

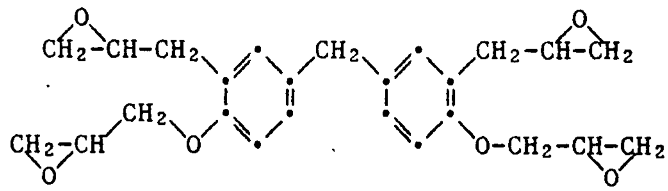

In einem 350 ml Sulfierkolben mit Rührer, Kühler und Tropftrichter werden 139,45 g (0,355 Mol) 3,3'-Diallyl-Bisphenol-F-4,4'-diglycidylether (Beispiel A2) in 20 ml $CHCl_3$ und 5,70 g Natriumacetat vorgelegt. Bei 30 °C werden innerhalb 5-6 Stunden 142,60 g (0,763 Mol) 40%ige Peressigsäure zugetropft. Nach Beendigen des Zutropfens wird das Reaktionsgemisch während ca. 30 Min. ausgerührt, anschliessend mit Wasser (2 x 500 ml) gewaschen und die organische Phase mit 20 g $Na_2SO_3$ versetzt und so lange gerührt, bis kein Peroxid mehr im Rekationsgemisch ist. Das Reaktionsgemisch wird nochmals mit Wasser gewaschen, über $Na_2SO_4$ getrocknet und eingeengt. Es resultieren 131,80 g (84,47 % d.Th.) eines hochviskosen Harzes vom Epoxidgehalt 7,66 Aeq./kg (81,31 %) und einer Viskosität von 2877 mPas/40 °C

IR (Film) 3500-3300, 2050-2800, 1740, 1610, 1500, 1250, 1130, 1040, 920, 830 $cm^{-1}$.

NMR($CDCl_3$) 2,5-3,0 m ~ 8H (O-$CH_2$-, arom.-$CH_2$); 3,0-3,5 m 4 H (-CH-$CH_2$); 3,5-4,5 m 8 H (-CH-$CH_2$) 6,6-7,3 d x d (J = 16 Hz) 6H (arom. H).

$\overline{M}_n$ 420; $\overline{M}_w$ 450.

B3) 3,3'-Diglycidyldiphenylmethan-2,2'-diglycidylether

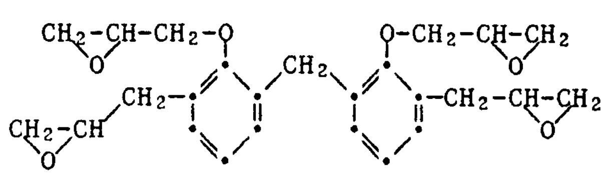

In einem 750 ml Sulfierkolben mit Rührer, Thermometer, Kühler und Tropftrichter werden 170,0 g (0,433 Mol) 3,3'-Diallyldiphenylmethan-2,2'-diglycidylether (Beispiel A3), 50 ml Chloroform und 6,98 g Natriumacetat vorgelegt. Bei ca. 30 °C werden innerhalb ca. 2 Stunden 174,06 g (0,919 Mol) 40%ige Peressigsäure zugetropft, wobei die Reaktion leicht exotherm ist. Nach Beendigen des Zutropfens wird das Reaktionsgemisch noch während 4 Stunden bei dieser Temperatur gehalten, anschliessend mit NaCl Lösung und Natriumsulfitlösung 10 % und Wasser gewaschen, über $Na_2SO_4$ getrocknet und eingeengt. Es resultieren 148,12 g (80,50 % d.Th.) des Tetraepoxids vom Epoxidgehalt 7,489 Aequ./kg (79,78 % d.Th.) und einer Viskosität von 2178 mPas/40 °C.

IR (Film) 3850, 3020-2960, 1460, 1250, 1090, 1020 $cm^{-1}$
$\overline{M}_n$ 374; $\overline{M}_w$ 387.

B4) 3,3'-Diglycidyldiphenylsulfon-4,4'-diglycidylether

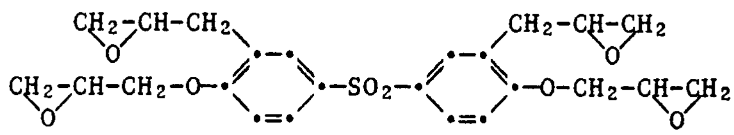

In einem 1,5 ℓ Sulfierkolben mit Rührer, Thermometer, Kühler und Tropftrichter werden 376,15 g (0,85 Mol) 3,3'-Diallyldiphenylsulfon-4,4'-diglycidylether (Beispiel A4), 13,80 g Natriumacetat und 380 ml Chloroform vorgelegt. Innerhalb 3-4 Stunden werden 370,06 g (1,80 Mol) 40%ige Peressigsäure bei 25-30 °C zugetropft, wobei sich das Reaktionsgemisch leicht erwärmt. Nach Beendigen des Zutropfens wird das Reaktionsgemisch noch während 8 Stunden bei dieser Temperatur ausreagieren gelassen, anschliessend wird das Reaktionsgemisch in Chloroform aufgenommen, mit 1 ℓ 2 % NaOH neutral gewaschen, die organische Phase abgetrennt und mit 250 g $Na_2SO_3$ peroxidfrei gemacht, filtriert und bei 60 °C am Vakuum eingeengt. Es resultieren 311,1 g (77,13 % d.Th.) eines viskosen Harzes vom Epoxidgehalt 6,040 Aeq./kg (71,65 %) und einer Viskosität 1245 mPas/80 °C.
IR (Film) 3500-3300, 3060-2950, 1750, 1600, 1490, 1300, 1250, 1100, 1020, 830 $cm^{-1}$.

NMR ($CDCl_3$) 2,5-3,5 m ~ 8H ($\overset{O}{\underline{CH_2}-CH}-$); 3,0-3,5 m 4H ($\overset{O}{CH_2-\underline{\underline{CH}}}-$);
3,5-4,5 m 8H ($OCH_2$ und Aryl-$CH_2$); 5,0-6,0 m (wenig restliches Allylgruppen-Edukt); 6,8-8,0 m 6H (Aryl-H).

$\overline{M}_n$ 450; $\overline{M}_w$ 481.

B5) 3,3'-Diglycidylbiphenyl-2,2'-diglycidylether

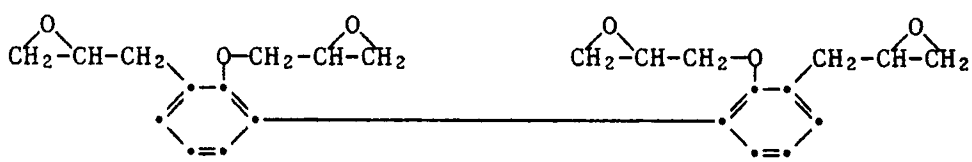

In einem 750 ml Sulfierkolben mit Rührer, Thermometer, Kühler und Tropftrichter werden 1892,24 g (0,50 Mol) 3,3'-Diallyl-2,2'-diglycidyloxybiphenyl (Beispiel A5) und 8,10 g Natriumacetat in 100 ml Chloroform gelöst. Bei einer Temperatur von 40-50 °C werden innerhalb 4-5 Stunden 217,77 g (1,061 Mol) 40%ige Peressigsäure zugetropft. Nach dem Zutropfen wird das Reaktionsgemisch noch während ca. einer Stunde ausreagieren gelassen. Anschliessend wird das Reaktionsgemisch mit 500 ml Chloroform verdünnt, mit 2 x 500 ml $H_2O$ und 250 ml $NaHCO_3$ neutral gewaschen, über $Na_2SO_4$ und $Na_2SO_3$ getrocknet bzw. peroxidfrei gemacht, filtriert und eingeengt. Es resultieren 196,83 g (95,83 %) eines leicht rötlichen, viskosen Harzes vom Epoxidgehalt 7,058 Aeq./kg (72,31 % d.Th.) und einer Viskosität von 5450 mPas/40 °C.
IR (Film) 3500-3300, 3060-2860, 1740, 1440, 1250, 1210, 1020 $cm^{-1}$
NMR ($CDCL_3$) 2,0-4,5 m 20H (Methylen- + Methin - H), 6,8 - 7,6 m 6H (arom. - H)
$\overline{M}_n$ 371: $\overline{M}_w$ 389.

C) Anwendungsbeispiele

C1) 100 Teile des gemäss Beispiel B4) hergestellten 3,3′-Diglycidyldiphenylsulfon-4,4′-diglycidylethers werden mit 29,9 Teilen Diaminodiphenylmethan vermischt.

Diese Mischung besitzt eine Ausgangsviskosität von 820 mPas (bei 80 °C; gemessen mit dem Epprecht-Viskosimeter).

Diese Mischung wird unter den folgenden Bedingungen gehärtet: 4 h bei 80 °C, 4 h bei 140 °C und 6 h bei 180 °C.

Die Biegefestigkeit einer derart ausgehärteten Probe beträgt 131,1 N/mm$^2$ (Messung gemäss DIN 53435).

Die Zugscherfestigkeit einer derart ausgehärteten Probe beträgt 6,5 N/mm$^2$ (Messung gemäss ISO 4587) und die Wasseraufnahme nach vier Tagen Lagerung bei Raumtemperatur beträgt 0,59 Gew.%.

C2) 100 Teile des gemäss Beispiel B1) hergestellten 3,3′-Diglycidylbiphenyl-4,4′-diglycidylethers und 40,7 Teile Diaminodiphenylmethan werden miteinander vermischt.

Die Ausgangsviskosität dieser Mischung beträft 130 mPas (bei 80 °C; gemessen mit dem Epprecht-Viskosimeter).

Diese Mischung wird wie in Beispiel C1) angegeben gehärtet.

Die Biegefestigkeit einer derart ausgehärteten Probe beträgt 108,4 N/mm$^2$ (Messung gemäss DIN 53435) und der Biegewinkel beträgt 36,4 °.

Die Zugscherfestigkeit einer derart ausgehäarteten Probe beträgt 6,2 N/mm$^2$ (Messung gemäss ISO 4587) und die Wasseraufnahme nach vier Tagen Lagerung bei Raumtemperatur beträgt 0,60 Gew.%.

**Patentansprüche**

**Patentansprüche für folgende Vertragsstaaten : CH, DE, FR, GB, IT, NL**

**1.** Verbindungen der Formel I

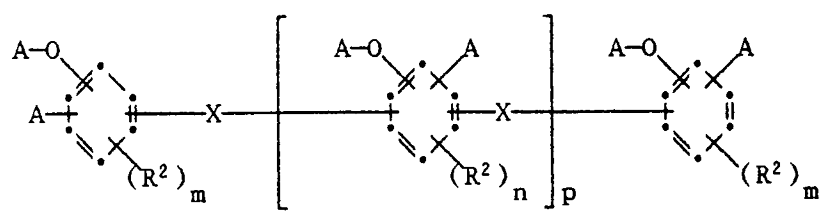

(I),

worin A eine Gruppe

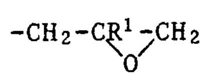

ist, R$^1$ Wasserstoff oder Methyl bedeutet, R$^2$ $C_1$-$C_6$ Alkyl, Halogen oder Phenyl ist,
X eine direkte C-C Bindung, -$CH_2$- oder -$SO_2$- darstellt,
m 0, 1, 2 oder 3 ist, n 0, 1 oder 2 bedeutet und
p 0 oder, im Falle von X = -$CH_2$-, auch eine ganze Zahl von 1 bis 6 bedeutet, mit der Massgabe, dass die Gruppen -A und -O-A jeweils in ortho-Position zueinander stehen.

**2.** Verbindungen der Formel I gemäss Anspruch 1, worin R$^1$ Wasserstoff ist.

**3.** Verbindungen der Formel I gemäss Anspruch 1, worin m und n 0 oder 1 sind.

**4.** Verbindungen der Formel I gemäss Anspruch 1, worin p 0 ist.

**5.** Verbindungen der Formel I gemäss Anspruch 1, worin m, n und p 0 sind.

6. Verbindungen der Formel I gemäss Anspruch 1, worin X eine direkte C-C Bindung oder $-SO_2-$ ist.

7. Verbindungen der Formel I gemäss Anspruch 1, worin die Reste -O-A jeweils in ortho- oder para-Position zur Brücke -X- stehen.

8. Verbindungen der Formel I gemäss Anspruch 7, worin die Reste -O-A jeweils in ortho-Position zur Brücke -X- stehen.

9. Verbindungen der Formel II

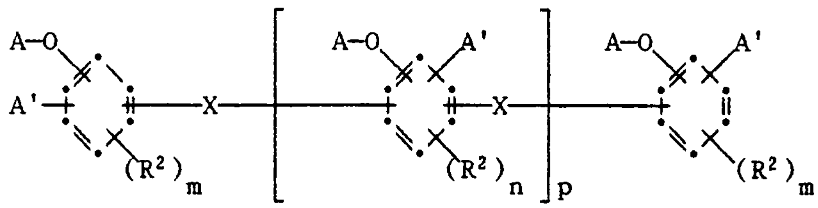

(II),

worin A eine Gruppe

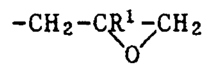

ist, $R^1$ Wasserstoff oder Methyl bedeutet, A' eine Gruppe $-CH_2-CR^1=CH_2$ ist, $R^2$ $C_1$-$C_6$Alkyl, Halogen oder Phenyl ist,
X eine direkte C-C Bindung, $-CH_2-$ oder $-SO_2-$ darstellt,
m 0, 1, 2 oder 3 ist, n 0, 1 oder 2 bedeutet und
p 0 oder, im Falle von X = $-CH_2-$, auch eine ganze Zahl von 1 bis 6 bedeutet, mit der Massgabe, dass die Gruppen -A' und -O-A jeweils in ortho-Position zueinander stehen.

10. Gemische enthaltend
a) ein Polyepoxid der Formel I gemäss Anspruch 1 und
b) einen Härter für Komponente a).

11. Verwendung der Gemische gemäss Anspruch 10 als Klebstoffe oder zur Herstellung von Verbundwerkstoffen und Laminaten.

**Patentansprüche für folgenden Vertragsstaat : ES**

1. Gemisch enthaltend
a) ein Polyepoxid der Formel I

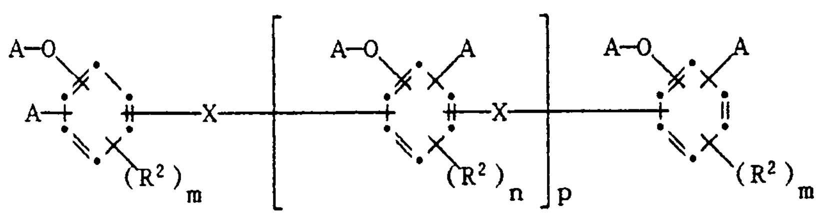

(I),

worin A eine Gruppe

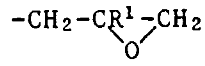

ist, $R^1$ Wasserstoff oder Methyl bedeutet, $R^2$ $C_1$-$C_6$Alkyl, Halogen oder Phenyl ist,
X eine direkte C-C Bindung, $-CH_2-$ oder $-SO_2-$ darstellt,
m 0, 1, 2 oder 3 ist, n 0, 1 oder 2 bedeutet und
p 0 oder, im Falle von X = $-CH_2-$, auch eine ganze Zahl von 1 bis 6 bedeutet, mit der Massgabe, dass die Gruppen -A und -O-A jeweils in ortho-Position zueinander stehen, und
b) einen Härter für Komponente a).

**2.** Gemische gemäss Anspruch 1, worin $R^1$ Wasserstoff ist.

**3.** Gemische gemäss Anspruch 1, worin m und n 0 oder 1 sind.

**4.** Gemische gemäss Anspruch 1, worin p 0 ist.

**5.** Gemische gemäss Anspruch 1, worin m, n und p 0 sind.

**6.** Gemische gemäss Anspruch 1, worin X eine direkte C-C Bindung oder $-SO_2-$ ist.

**7.** Gemische gemäss Anspruch 1, worin die Reste -O-A jeweils in ortho- oder para-Position zur Brücke -X- stehen.

**8.** Gemische gemäss Anspruch 7, worin die Reste -O-A jeweils in ortho-Position zur Brücke -X- stehen.

**9.** Verfahren zur Herstellung von Verbindungen der Formel I gemäss Anspruch 1, dadurch gekennzeichnet, dass man Verbindungen der Formel II einsetzt

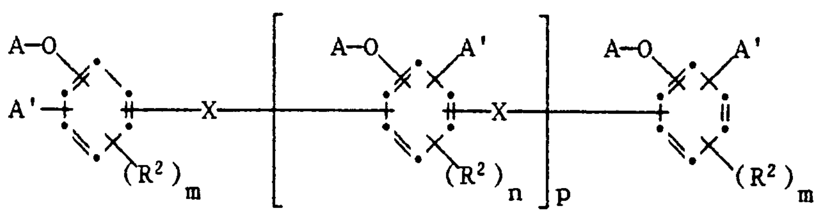

(II),

worin A eine Gruppe

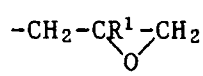

ist, $R^1$ Wasserstoff oder Methyl bedeutet, A′ eine Gruppe $-CH_2-CR^1=CH_2$ ist, $R^2$ $C_1$-$C_6$Alkyl, Halogen oder Phenyl ist,
X eine direkte C-C Bindung, $-CH_2-$ oder $-SO_2-$ darstellt,
m 0, 1, 2 oder 3 ist, n 0, 1 oder 2 bedeutet und
p 0 oder, im Falle von X = $-CH_2-$, auch eine ganze Zahl von 1 bis 6 bedeutet, mit der Massgabe, dass die Gruppen -A′ und -O-A jeweils in ortho-Position zueinander stehen, und besagte Verbindungen der Formel II durch Behandlung mit Persäure epoxidiert.

**10.** Verfahren zur Herstellung von Verbindungen der Formel I gemäss Anspruch 1, dadurch gekennzeichnet, dass man Verbindungen der Formel IIa einsetzt

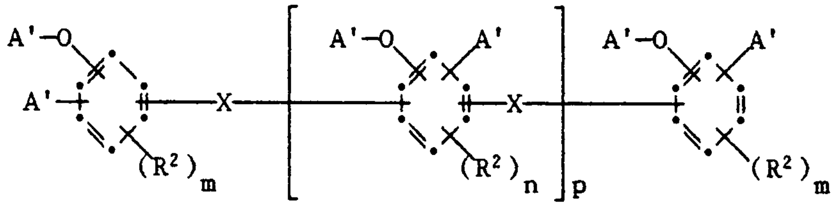

(IIa),

worin A′, $R^2$, X, m, n und p die in Anspruch 9 gegebene Bedeutung besitzen und die Gruppen -A′ und -O-A jeweils in ortho-Position zueinander stehen, und besagte Verbindungen der Formel IIa durch Behandlung mit Persäure epoxidiert.

**11.** Verfahren zur Herstellung von Verbindungen der Formel II gemäss Anspruch 9, dadurch gekennzeichnet, dass man Allylphenole der Formell IIb einsetzt

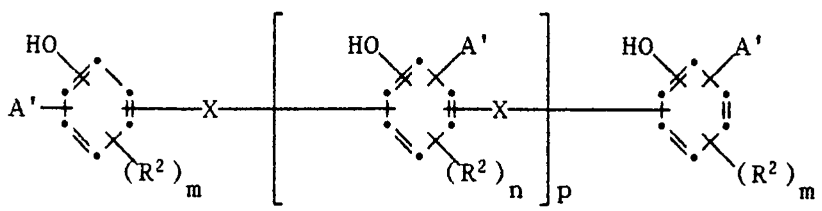

(IIb),

worin A′, $R^2$, X, n, m und p die in Anspruch 9 angegebenen Bedeutungen besitzen und die Reste -A′ und -OH jeweils in ortho-Position zueinander stehen, mit Epihalogenhydrin oder $\beta$-Methylepihalogenhydrin umsetzt.

**12.** Verwendung der Gemische gemäss Anspruch 1 als Klebstoffe oder zur Herstellung von Verbundwerkstoffen und Laminaten.

**Claims**
**Claims for the following Contracting States : CH, DE, FR, GB, IT, NL**

**1.** A compound of the formula I

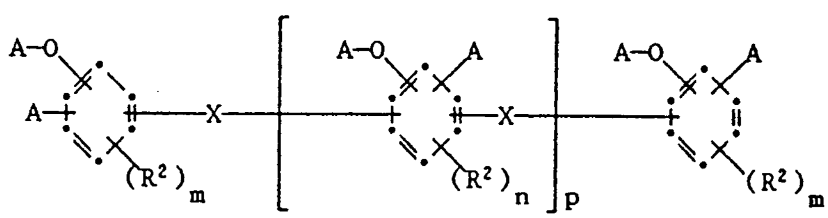

(I),

in which A is a group

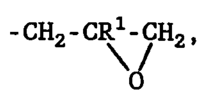

$R^1$ is hydrogen or methyl,
$R^2$ is $C_1$-$C_6$alkyl, halogen or phenyl,
X is a direct C-C bond, $-CH_2-$ or $-SO_2-$,
m is 0, 1, 2 or 3, n is 0, 1 or 2 and
p is 0 or, in the case of X = $-CH_2-$, is also an integer from 1 to 6, with the proviso that the groups -A

and -O-A are always in the ortho-position relative to one another.

**2.** A compound of the formula I according to claim 1, wherein $R^1$ is hydrogen.

**3.** A compound of the formula I according to claim 1, wherein m and n are 0 or 1.

**4.** A compound of the formula I according to claim 1, wherein p is 0.

**5.** A compound of the formula I according to claim 1, wherein m, n and p are 0.

**6.** A compound of the formula I according to claim 1, wherein X is a direct C-C bond or $-SO_2-$.

**7.** A compound of the formula I according to claim 1, wherein the radicals -O-A are always in the ortho-position or para-position relative to the -X- bridge.

**8.** A compound of the formula I according to claim 7, wherein the radicals -O-A are always in the ortho-position relative to the -X- bridge.

**9.** A compound of the formula II

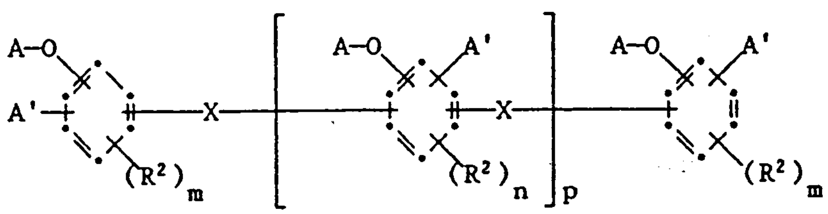

(II),

in which A is a group

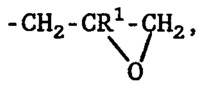

$R^1$ is hydrogen or methyl,
A' is a group $-CH_2-CR^1-CH_2$, $R^2$ is $C_1$-$C_6$alkyl, halogen or phenyl,
X is a direct C-C bond, $-CH_2-$ or $-SO_2-$,
m is 0, 1, 2 or 3, n is 0, 1 or 2 and
p is 0 or, in the case of X = $-CH_2-$, is also an integer from 1 to 6, with the proviso that the groups -A' and -O-A are always in the ortho-position relative to one another.

**10.** A mixture comprising
a) a polyepoxide of the formula I according to claim 1 and
b) a curing agent for component a).

**11.** The use of a mixture according to claim 10 as an adhesive or for the production of a composite material or laminate.

**Claims for the following Contracting State : ES**

**1.** A mixture comprising
a) a polyepoxide of the formula I

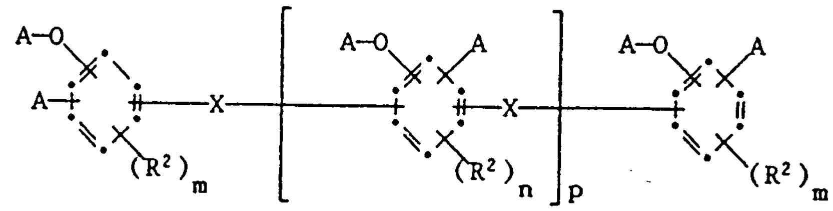

(I),

in which A is a group

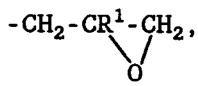

$R^1$ is hydrogen or methyl,
$R^2$ is $C_1$-$C_6$alkyl, halogen or phenyl,
X is a direct C-C bond, $-CH_2-$ or $-SO_2-$,
m is 0, 1, 2 or 3, n is 0, 1 or 2 and
p is 0 or, in the case of X = $-CH_2-$, is also an integer from 1 to 6, with the proviso that the groups -A and -O-A are always in the ortho-position relative to one another, and
b) a curing agent for component a).

**2.** A mixture according to claim 1, wherein $R^1$ is hydrogen.

**3.** A mixture according to claim 1, wherein m and n are 0 or 1.

**4.** A mixture according to claim 1, wherein p is 0.

**5.** A mixture according to claim 1, wherein m, n and p are 0.

**6.** A mixture according to claim 1, wherein X is a direct C-C bond or $-SO_2-$.

**7.** A mixture according to claim 1, wherein the radicals -O-A are always in the ortho-position or para-position relative to the -X- bridge.

**8.** A mixture according to claim 7, wherein the radicals -O-A are always in the ortho-position relative to the -X- bridge.

**9.** A process for the preparation of a compound of the formula I according to claim 1, which comprises employing a compound of the formula II

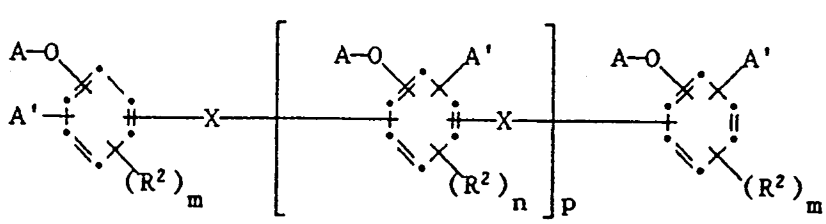

(II),

in which A is a group

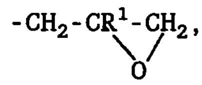

$R^1$ is hydrogen or methyl,
A' is a group $-CH_2-CR^1=CH_2$, $R^2$ is $C_1$-$C_6$alkyl, halogen or phenyl,
X is a direct C-C bond, $-CH_2-$ or $-SO_2-$,
m is 0, 1, 2 or 3, n is 0, 1 or 2 and
p is 0 or, in the case of X = $-CH_2-$, is also an integer from 1 to 6, with the proviso that the groups -A' and -O-A are always in the ortho-position relative to one another, and epoxidising said compound of the formula II by treatment with a peracid.

**10.** A process for the preparation of a compound of the formula I according to claim 1, which comprises employing a compound of the formula IIa

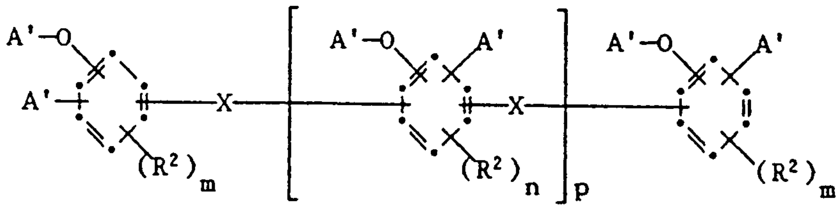

(IIa),

in which $A^1$, $R^2$, X, m, n and p have the meanings given in claim 9 and the groups -A' and -O-A are always in the ortho-position relative to one another, and epoxidising said compound of the formula IIa by treatment with a peracid.

**11.** A process for the preparation of a compound of the formula II according to claim 9, which comprises employing an allylphenol of the formula IIb

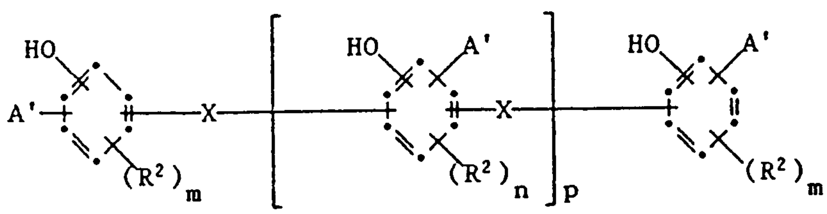

(IIb),

in which A', $R^2$, X, n, m and p have the meanings given in claim 9 and the radicals -A' and -OH are always in the ortho-position relative to one another, with an epihalohydrin or a $\beta$-methylepihalohydrin.

**12.** The use of a mixture according to claim 1 as an adhesive or for the production of a composite material or laminate.

**Revendications**
**Revendications pour les Etats contractants suivants : CH, DE, FR, GB, IT, NL**

**1.** Composés répondant à la formule I :

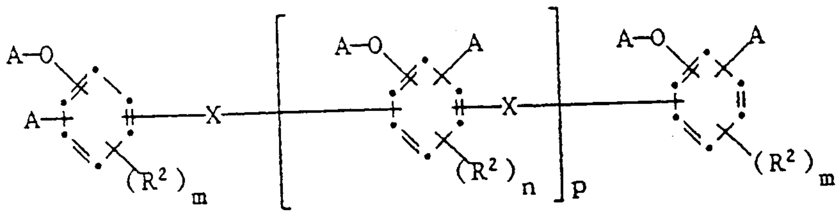

(I),

dans laquelle

A représente un radical

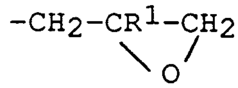

dans lequel $R^1$ désigne l'hydrogène ou un méthyle,

$R^2$ représente un alkyle en $C_1$-$C_6$ un halogène ou un phényle,

X représente une liaison directe C-C, un radical $-CH_2-$ ou un radical $-SO_2-$,

m est égal à 0, à 1, à 2 ou à 3,

n est égal à 0, à 1 ou à 2 et

p est égal à 0 ou, dans le cas où X représente $-CH_2-$, il représente également un nombre entier de 1 à 6,

avec la condition que les radicaux -A et -O-A se trouvent, à chaque fois, en position ortho l'un par rapport à l'autre.

**2.** Composés de formule I selon la revendication 1 dans lesquels $R^1$ représente l'hydrogène.

**3.** Composés de formule I selon la revendication 1 dans lesquels m et n sont égaux à 0 ou à 1.

**4.** Composés de formule I selon la revendication 1 dans lesquels p est égal à 0.

**5.** Composés de formule I selon la revendication 1 dans lesquels m, n et p sont égaux à 0.

**6.** Composés de formule I selon la revendication 1 dans lesquels X représente une liaison directe C-C ou un radical $-SO_2-$.

**7.** Composés de formule I selon la revendication 1 dans lesquels les radicaux -O-A sont à chaque fois en position ortho ou para relativement au pont -X-.

**8.** Composés de formule I selon la revendication 7 dans lesquels les radicaux -O-A sont à chaque fois en ortho par rapport au pont -X-.

**9.** Composés répondant à la formule II :

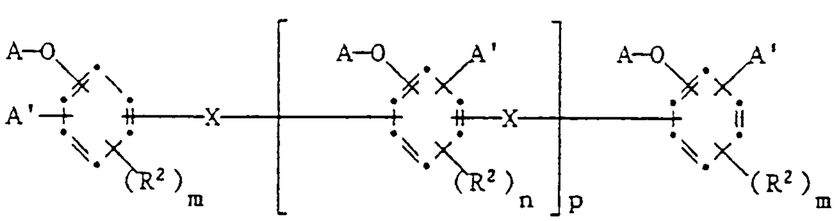

(II),

dans laquelle

A représente un radical

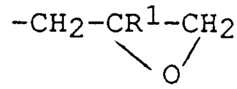

dans lequel $R^1$ désigne l'hydrogène ou un méthyle,

A' représente un radical $-CH_2-CR^1=CH_2$,

$R^2$ représente un alkyle en $C_1$-$C_6$, un halogène ou un phényle,

X représente une liaison directe C-C, $-CH_2-$ ou $-SO_2-$,

m est égal à 0, à 1, à 2 ou à 3,

n est égal à 0, à 1 ou à 2 et

p est égal à 0 ou, lorsque X représente un radical $-CH_2-$, il peut aussi représenter un nombre entier de 1 à 6,

avec la condition que les radicaux -A' et -O-A se trouvent, à chaque fois, en position ortho l'un par rapport à l'autre.

**10.** Mélanges contenant :
a) un polyépoxyde de formule I selon la revendication 1 et
b) un durcisseur pour la composante a).

**11.** Application des mélanges selon la revendication 1 comme colles ou pour la fabrication de matériaux composites et de stratifiés.

**Revendications pour l'Etat contractant suivant : ES**

**1.** Mélanges contenant :
a) un polyépoxyde répondant à la formule I :

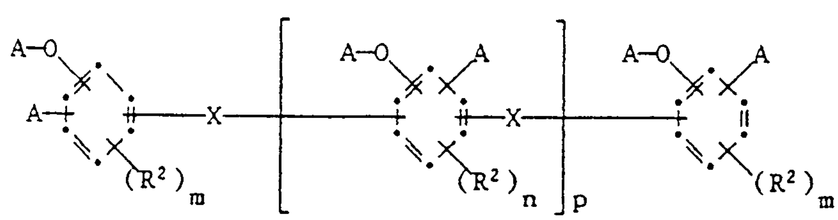 (I),

dans laquelle

A représente un radical

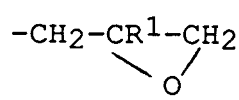

dans lequel $R^1$ désigne l'hydrogène ou un méthyle,

$R^2$ représente un alkyle en $C_1$-$C_6$, un halogène ou un phényle,

X représente une liaison directe C-C, un radical $-CH_2-$ ou un radical $-SO_2-$,

m est égal à 0, à 1, à 2 ou à 3,

n est égal à 0, à 1 ou à 2 et

p est égal à 0 ou, dans le cas où X représente $-CH_2-$, il représente également un nombre entier de 1 à 6,

avec la condition que les radicaux -A et -O-A se trouvent, à chaque fois, en position ortho l'un par rapport à l'autre, et

b) un durcisseur pour la composante a).

2. Mélanges selon la revendication 1 dans lesquels $R^1$ représente l'hydrogène.

3. Mélanges selon la revendication 1 dans lesquels m et n sont égaux à 0 ou à 1.

4. Mélanges selon la revendication 1 dans lesquels p est égal à 0.

5. Mélanges selon la revendication 1 dans lesquels m, n et p sont égaux à 0.

6. Mélanges selon la revendication 1 dans lesquels X représente une liaison directe C-C ou un radical $-SO_2-$.

7. Mélanges selon la revendication 1 dans lesquels les radicaux -O-A se trouvent, à chaque fois, en position ortho ou para relativement au pont -X-.

8. Mélanges selon la revendication 1 dans lesquels les radicaux -O-A se trouvent, à chaque fois, en ortho par rapport au pont -X-.

9. Procédé pour préparer des composés de formule I selon la revendication 1, procédé caractérisé en ce qu'on époxyde, en les traitant par un peracide, des composés répondant à la formule II :

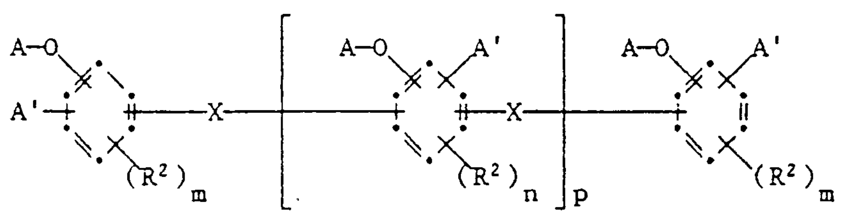

(II),

dans laquelle

A représente un radical

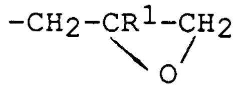

dans lequel $R^1$ désigne l'hydrogène ou un méthyle,

A' représente un radical $-CH_2-CR^1=CH_2$,

$R^2$ représente un alkyle en $C_1$-$C_6$, un halogène ou un phényle,

X représente une liaison directe C-C, $-CH_2-$ ou $-SO_2-$,

m est égal à 0, à 1, à 2 ou à 3,

n est égal à 0, à 1 ou à 2 et

p est égal à 0 ou, lorsque X représente un radical $-CH_2-$, il peut aussi représenter un nombre entier dé 1 à 6,

avec la condition que les radicaux -A' et -O-A se trouvent, à chaque fois, en position ortho l'un par rapport à l'autre.

10. Procédé pour préparer des composés de formule I selon la revendication 1, procédé caractérisé en ce qu'on époxyde, en les traitant par un peracide, des composés répondant à la formule IIa :

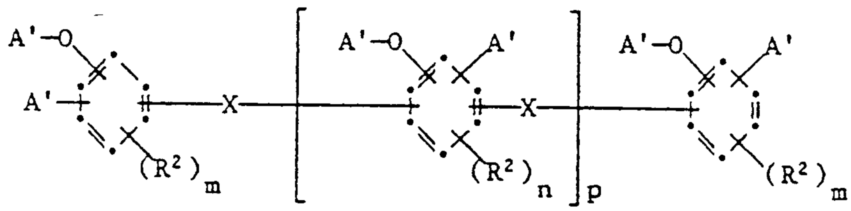

(IIa),

dans laquelle A', $R^2$, X, m, n et p ont les significations qui leur ont été données à la revendication 9 et les radicaux -A' et -O-A' se trouvent, à chaque fois, en position ortho l'un par rapport à l'autre.

**11.** Procédé pour préparer des composés de formule II selon la revendication 9, procédé caractérisé en ce qu'on fait réagir des allylphénols répondant à la formule IIb :

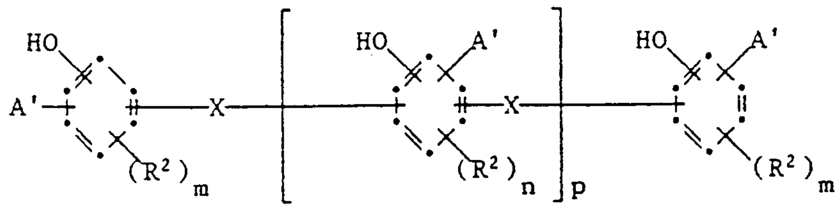

(IIb)

dans laquelle A', $R^2$, X, m, n et p ont les significations qui leur ont été données à la revendication 9 et les radicaux -A' et -OH se trouvent, à chaque fois, en position ortho l'un par rapport à l'autre,
avec une épihalogénhydrine ou une $\beta$-méthylépihalogénhydrine.

**12.** Application des mélanges selon la revendication 1 comme colles ou pour la fabrication de matériaux composites et de stratifiés.